# EUROPEAN PATENT APPLICATION

(11) **EP 3 835 788 A1**
(43) Date of publication of application: **16.06.2021**
(21) Application number: 19846491.9
(22) Date of filing: 05.08.2019
(51) Int. Cl.: G01N 33/569, G01N 33/543

(54) **METHOD FOR IMMUNOLOGICALLY DETECTING MYCOPLASMA PNEUMONIAE**

(30) Priority: 06.08.2018 JP 2018147371
(71) Applicant: Sekisui Medical Co., Ltd., Tokyo 103-0027 (JP)
(72) Inventor: OCHIAI Yasushi, Tokyo 103-0027 (JP); NAKAMURA Tomofumi, Tokyo 103-0027 (JP)
(74) Representative: Hoffmann Eitle
(86) International application number: PCT/JP2019/030629
(87) International publication number: WO 2020/031931

(57) **Abstract**

An object of the present invention is to provide an immunological detection method comprising carrying out an immunoreaction using an antibody to *Mycoplasma pneumoniae* protein P30, in which *Mycoplasma pneumoniae* is detected with high sensitivity. Provided is an immunological detection method for *Mycoplasma pneumoniae* using an antibody to *Mycoplasma pneumoniae* protein P30, the method comprising carrying out an immunoreaction in the presence of a polyoxyethylene alkyl amine. The immunological detection method that uses immunochromatography is also provided.

## Description

### TECHNICAL FIELD

The present invention relates to a method of immunologically detecting *Mycoplasma pneumoniae.* In particular, the present invention relates to a method of immunologically detecting *Mycoplasma pneumoniae* using an antibody to *Mycoplasma pneumoniae* protein P30.

### BACKGROUND ART

*Mycoplasma pneumonia* is a respiratory infectious disease caused by a pathogenic bacterium, *Mycoplasma pneumoniae.* The prevalence is higher in children, and children younger than 4 years old account for 30% of the patients and children younger than 9 years old account for 60% or more.

Many patients of *Mycoplasma pneumonia* merely suffer from bronchitis and the like, but some patients may advance in severity to be accompanied with pneumonia or other complications. Accordingly, rapid definitive diagnosis and selection of an appropriate antimicrobial are important, and thus immunochromatography which is simple in operation and which enables rapid detection is suitable.

For example, Patent Document 1 discloses an immunochromatography apparatus using a monoclonal antibody specific to *Mycoplasma pneumoniae* protein P30.

In addition, Patent Document 2 disclose an immunochromatography apparatus using an antibody specific to *Mycoplasma pneumoniae* ribosomal protein L7/L12.

Incidentally, when an antibody is used for detection, bacterial cells or proteins to be detected are sometimes pretreated with a surfactant or the like to expose the site detected by the antibody before the detection.

Patent Document 1 discloses a kit in which a specimen diluent containing a nonionic surfactant having an HLB value of 13 to 17 is used with the immunochromatography apparatus. However, while there are a huge number of nonionic surfactants having an HLB value of 13 to 17, two examples of a 1:1 mixture of polyoxyethylene octylphenyl ether (triton X-100) and polyoxyethylene sorbitan monooleate (tween 20) and a 1:1 mixture of polyoxyethylene octylphenyl ether (Nonidet p-40) and a polyoxyethylene/polyoxypropylene alkyl ether (Nonion MN-811) are the only nonionic surfactants that are actually described in Examples, and whether the huge number of other nonionic surfactants having an HLB value of 13 to 17 can be used is not described.

In addition, according to a test by the present inventors, none of polyoxyethylene octylphenyl ether (triton X-100), polyoxyethylene sorbitan monooleate (tween 20), and polyoxyethylene octylphenyl ether (Nonidet p-40) as described above provided a sufficient sensitivity in the detection using a *Mycoplasma pneumoniae* P30 monoclonal antibody.

Patent Document 2 discloses a kit in which a specimen diluent containing a polyoxyethylene alkyl ether or a nonionic surfactant having an HLB value of 12 to 15 is used with an immunochromatography apparatus. However, polyoxyethylene cetyl ether (Brij 58) and a synthetic alcohol EO adduct (LEOCOL TD90, 120) are the only nonionic surfactants that are actually described in Examples, and a huge number of other nonionic surfactants having an HLB value of 12 to 15 are not disclosed. In addition, an antibody to *Mycoplasma pneumoniae* ribosome protein L7/L12 is the only antibody tested, and any effect of the nonionic surfactants on an antibody to P30 protein is not disclosed nor suggested.

In addition, according to a test by the present inventors, in the detection using a *Mycoplasma pneumoniae* P30 monoclonal antibody, a sufficient sensitivity is not provided in either case where polyoxyethylene cetyl ether (Brij 58) or a synthetic alcohol EO adduct (LEOCOL TD90, 120) is added to a specimen diluent.

Thus, a technique for highly sensitive detection still remains to be established in a method of immunologically detecting *Mycoplasma pneumoniae* using an antibody to *Mycoplasma pneumoniae* protein P30.

### CITATION LIST

### PATENT LITERATURE

Patent Document 1: JP 2017-009571 A
patent Document 2: JP 2014-167439 A

### SUMMARY OF INVENTION

### TECHNICAL PROBLEM

An object of the present invention is to provide an immunological detection method comprising carrying out an immunoreaction using an antibody to *Mycoplasma pneumoniae* protein P30, in which *Mycoplasma pneumoniae* is detected with high sensitivity.

### SOLUTION TO PROBLEM

As a result of intensive and extensive studies for achieving the above object, the present inventors have found that when an antibody to *Mycoplasma pneumoniae* protein P30 is used in the presence of a polyoxyethylene alkyl amine among many surfactants to carry out an immunoreaction, *Mycoplasma pneumoniae* can be detected with high sensitivity, thus completing the present invention.

Specifically, the present invention has the following configuration.
<1> An immunological detection method for *Mycoplasma pneumoniae* using an antibody to *Mycoplasma pneumoniae* protein P30, wherein an immunoreaction is carried out in the presence of a polyoxyethylene alkyl amine.
<2> The detection method according to <1>, wherein the immunological detection method is an immunochromatographic detection method.
<3> The method according to <1> or <2>, wherein the immunochromatographic detection method includes the following steps (A) to (C):
   (A) a step of mixing a sample containing *Mycoplasma pneumoniae* and a sample diluent containing a polyoxyethylene alkyl amine;
   (B) a step of supplying the mixture obtained in the above (A) to the sample-supply portion of the test strip defined below,
   the test strip comprising:
   a membrane of a porous body having at least a sample-supply portion, a development portion, and a detection portion,
   a conjugate containing a first antibody to *Mycoplasma pneumoniae* protein P30 labeled with a labeling substance retained in a part of the development portion in a dissoluble state, and
   the detection portion that is located in a part of the development portion on the downstream side of the conjugate-holding portion and having a second antibody to *Mycoplasma pneumoniae* protein P30 immobilized thereon; and
   (C) a step of detecting in the detection portion a complex of *Mycoplasma pneumoniae* in the sample and the conjugate.
<4> A *Mycoplasma pneumoniae* detection kit comprising the following configurations (1) and (2):
   (1) a detection reagent using an antibody to *Mycoplasma pneumoniae* protein P30; and
   (2) a sample diluent containing a polyoxyethylene alkyl amine.
<5> The detection kit according to <4>, wherein the detection reagent using an antibody to *Mycoplasma pneumoniae* protein P30 in the above (1) is the following configuration (1)':
   (1)' an immunochromatographic test strip comprising a membrane of a porous body having at least a sample-supply portion, a development portion, and a detection portion,
   a conjugate containing a first antibody to *Mycoplasma pneumoniae* protein P30 labeled with a labeling substance retained in a part of the development portion in a dissoluble state, and
   the detection portion that is located in a part of the development portion on the downstream side of the conjugate-holding portion and has a second antibody to *Mycoplasma pneumoniae* protein P30 immobilized thereon.
<6> A sample diluent containing a polyoxyethylene alkyl amine for use in an immunological detection method that uses an antibody to *Mycoplasma pneumoniae* protein P30.
<7> A sample pretreatment method for immunologically detecting *Mycoplasma pneumoniae,* which comprises a step of bringing a polyoxyethylene alkyl amine into contact with a sample containing *Mycoplasma pneumoniae.*

### ADVANTAGEOUS EFFECTS OF INVENTION

In the present invention, an antibody to *Mycoplasma pneumoniae* protein P30 is used in the presence of a polyoxyethylene alkyl amine to carry out an immunoreaction, whereby *Mycoplasma pneumoniae* can be detected with high sensitivity.

### BRIEF DESCRIPTION OF DRAWING

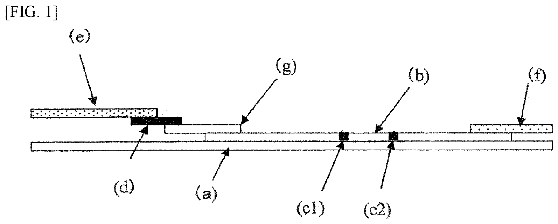

[Fig. 1] It is a schematic diagram of the configuration of a test strip of the present invention.

### DESCRIPTION OF EMBODIMENTS

### (Sample)

In the present invention, a sample means a specimen that was taken from a patient and that potentially contains *Mycoplasma pneumoniae* which is to be detected.

A patient specimen may be used as it is as a sample, or may be appropriately diluted with a sample diluent and then used as a sample. Alternatively, a patient specimen may be appropriately diluted and filtrated, and then used as a sample. Note that a sample diluent is sometimes referred to as a specimen diluent, a sample extraction solution, a sample development solution, a sample suspender, or the like, which are used as synonyms.

### (Polyoxyethylene Alkyl Amine)

The immunological detection method of the present invention is characterized by comprising carrying out an immunoreaction in the presence of a polyoxyethylene alkyl amine. A polyoxyethylene alkyl amine is a weak cationic compound represented by the following general formula, and has such a nature that, as the number of oxyethylene groups increases, the nonionic nature increases and the affinity to water increases (HLB increases).

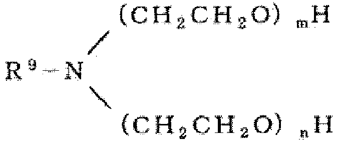

A polyoxyethylene alkyl amine for the present invention preferably has an HLB of about 5.0 to 18.0, more preferably 6.0 to 16.0, still more preferably 7.0 to 12.0, and most preferably 9.0 to 11.0 in view of the solubility in water. Specific examples thereof include products sold under trade names of AMIET (registered trademark, the same applies hereinafter) 102 (HLB 6.3), AMIET 105 (HLB 9.8), AMIET 105A (HLB 10.8), AMIET 302 (HLB 5.1), AMIET 320 (HLB 15.4) (Kao Corporation), LIPONOL (registered trademark) series (Lion Specialty Chemicals Co., Ltd.), BLAUNON series (AOKI OIL INDUSTRIAL Co., Ltd.), and IONET (registered trademark) EP-300S (SANYO CHEMICAL INDUSTRIES, LTD.).

The concentration of the polyoxyethylene alkyl amine in an immunoreaction system is preferably 0.01% to 10.0%, more preferably 0.1% to 5%, still more preferably 0.2% to 3%, and most preferably 0.3 to 1.5%.

In an aspect of incorporating the polyoxyethylene alkyl amine in a reaction system, the polyoxyethylene alkyl amine may be directly added to an immunoreaction system. In a typical aspect, however, the polyoxyethylene alkyl amine added in advance to a sample diluent is brought into contact with an antibody to *Mycoplasma pneumoniae* protein P30, thereby incorporating the polyoxyethylene alkyl amine in an immunoreaction system, as described later.

### (Sample Diluent)

The sample diluent of the present invention contains the polyoxyethylene alkyl amine, and preferably further contains a buffer.

The concentration of the polyoxyethylene alkyl amine in the sample diluent of the present invention is preferably in the range of 0.01% to 10.0%, more preferably 0.1% to 5%, still more preferably 0.2% to 3%, and most preferably 0.3 to 1.5%.

Examples of the buffer contained in the sample diluent of the present invention include a phosphate buffer, Tris buffer, and Good's buffer which are generally used. The sample diluent of the present invention may be any one that contains the above compound and a buffer solution. The sample diluent may be a buffer solution containing the above compound. The sample diluent may further contain a salt, such as sodium chloride, stabilizers or preservative such as sucrose, an antiseptic, such as ProClin (registered trademark), or the like. The salt includes, in addition to a salt that is incorporated for adjusting the ionic strength, such as sodium chloride, a salt that is added for adjusting the pH of the buffer, such as sodium hydroxide. The sample diluent of the present invention preferably has a pH in the range of 6.0 to 10.0, more preferably 7.0 to 9.0, and still more preferably 7.5 to 8.5.

Since the sample diluent of the present invention is liquid for diluting a sample, the sample diluent plays a roll of dissolving, extracting, or dispersing a sample (specimen) containing *Mycoplasma pneumoniae* added to the sample diluent to dilute the sample. Accordingly, a sample and the sample diluent are typically in a mixed state at a time when the sample is supplied to a sample pad of a test strip. However, in addition to the above aspect, the present invention includes an aspect where a sample is directly supplied to a sample pad or a sample diluted in a diluent other than the sample diluent of the present invention is supplied to a sample pad and the sample diluent of the present invention is simultaneously or subsequently supplied to the sample pad.

The immunological detection method of the present invention may be any detection method that uses an immunoreaction, and examples thereof include a latex immunoagglutination assay (hereinafter referred to as LTIA method) which is a particle agglutination immunoassay, an ELISA method which is a typical sandwich immunoassay method and is also a labeled immunoassay, and a detection method using immunochromatography. Among them, a detection method using immunochromatography is particularly desirable. Examples of the detection method using immunochromatography include a flow through-based and a lateral flow-based immunochromatography, and a detection method using a lateral flow-based immunochromatography is desirable due to the simplicity and rapidity.

When the sample diluent of the present invention is used in the LTIA method, after diluting a sample with the sample diluent of the present invention, the dilution is mixed with a latex reagent, whereby an immunoagglutination reaction can be detected. When the latex reagent consists of a first reagent containing a buffer and a second reagent containing a latex reagent, the specimen diluent of the present invention can be used as the first reagent.

The present invention also provides a sample pretreatment method for immunologically detecting *Mycoplasma pneumoniae,* the method comprising a step of bringing a polyoxyethylene alkyl amine into contact with a sample containing *Mycoplasma pneumoniae.*

Typically, a pretreatment can be performed by incorporating a polyoxyethylene alkyl amine in a specimen diluent and mixing the specimen diluent with a sample.

### (Substance to be Detected)

The substance to be detected (analyte) of the present invention is *Mycoplasma pneumoniae.* In the present invention, the presence of *Mycoplasma pneumoniae* is detected or quantified directly by detecting *Mycoplasma pneumoniae* protein P30 using an antibody to *Mycoplasma pneumoniae* protein P30.

### (Detection Using Immunochromatography)

The immunochromatographic test strip of the present invention is a test strip for detecting a complex of a substance to be detected in a sample and a conjugate by developing the sample in an insoluble membrane. The test strip includes a membrane of a porous body having (1) a sample-supply portion, (2) a development portion, and (3) a detection portion, a part of the development portion holding in a dissoluble state a conjugate containing a first antibody labeled with a labeling substance, the detection portion being present in a part of the development portion on the downstream side of the conjugate-holding portion and having a second antibody immobilized thereon.

In an aspect of the present invention, the polyoxyethylene alkyl amine is contained in the sample diluent. Another aspect is also possible in which a part of pads constituting the test strip is impregnated with the polyoxyethylene alkyl amine. For example, a sample pad, a third pad, a conjugate pad, an insoluble membrane, or the like is impregnated with the polyoxyethylene alkyl amine. Still another aspect is also possible in which a filter chip is impregnated with the polyoxyethylene alkyl amine in the present invention.

In the conjugate, an antibody that immunologically reacts with a substance to be detected is immobilized to a labeling body, and regarding the manner of presence of the conjugate, the conjugate may be present as a conjugate pad or the conjugate may be present in a state that a pad other than a sample pad, a third pad, and an insoluble membrane is impregnated with the conjugate (type A), or the conjugate may be present as a conjugate section in a part of a sample pad (type B), or the conjugate may be present as an individual conjugate reagent other than the test strip in a manner that the conjugate can be mixed with a specimen (type C).

A test strip in which a conjugate is present in a manner of the type A will be described below.

A sample pad, a conjugate pad, a third pad, and an insoluble membrane are disposed in this order from upstream toward downstream in the sample flowing direction so that each pad at least partially overlaps with the layer above and the layer below. An example of the test strip having such an arrangement is shown in Fig. 1.

When a sample containing a substance to be detected is supplied to the sample pad in such a test strip, the substance to be detected flows through the sample pad toward the conjugate pad on the downstream side. In the conjugate pad, the substance to be detected flows through the conjugate pad while coming in contact with the conjugate to form a complex (aggregate). Then, the complex flows through the porous third pad disposed in contact with the lower surface of the conjugate pad to develop into the insoluble membrane.

Since an antibody that immunologically reacts with the substance to be detected is immobilized on a part of the insoluble membrane, the complex is to be immobilized here by bonding thereto though an immunoreaction. The immobilized complex is detected by a means for detecting the absorbance, reflected light, fluorescence, or magnetism due to the conjugate.

Next, a test strip in which the conjugate is present in a manner of the type B will be described.

It differs from the test strip of the type A in that a sample pad and a conjugate pad are integrated, that is, a sample-supply portion and a conjugate section are configured in a part of a sample pad.

The sample-supply portion is a section where a sample containing a substance to be detected is to be supplied, and the conjugate section is a section containing a conjugate. The sample-supply portion is on the upstream side of the conjugate section.

Next, a test strip in which the conjugate is present in a manner of the type C will be described.

It differs from the test strip of the type A in that there is no conjugate pad, and a conjugate is present as an individual conjugate reagent. An example is a filter chip in which a conjugate is contained in a filter. When such a filter chip is used and a sample diluent and a substance to be detected are allowed to pass through the filter chip, the conjugate binds to the substance to be detected to form a complex (aggregate). By supplying the complex to the same test strip as that of the type A except for having no conjugate pad, the substance to be detected can be detected.

Besides the above, the conjugate pad or the filter chip can contain the polyoxyethylene alkyl amine in the present invention.

### (Sample Pad)

A sample pad used in the present invention is a section to receive a sample and includes any substances and forms that can absorb a liquid sample and allow the liquid and the object to be detected to pass through in a state of being molded into a pad shape. Specific examples of materials suitable for a sample pad include, but not limited to, glass fiber, acrylic fiber, a hydrophilic polyethylene material, dry paper, paper pulp, and a textile. A glass fiber pad is suitably used. The sample pad may also have a function of a conjugate pad as described later. In addition, the sample pad can contain a blocking reagent which is generally used for the purpose of preventing or suppressing a nonspecific reaction (adsorption) in an antibody-immobilized membrane.

Besides the above, the sample pad can also contain the polyoxyethylene alkyl amine in the present invention.

### (Third pad)

A third pad is desirably provided as required depending on the nature of the sample or the like, and any type that can allow a complex of the substance to be detected in the sample and the conjugate to pass through the pad can be used.

The third pad of the present invention has an average pore size of, for example, 1 to 100 µm, preferably 5 to 80 µm, more preferably 10 to 60 µm, further preferably 15 to 55 µm, particularly preferably 20 to 50 µm, and most preferably 25 to 45 µm.

Besides the above, the third pad can contain the polyoxyethylene alkyl amine in the present invention.

### (Insoluble membrane)

The insoluble membrane used in the present invention has at least one detection portion on which an antibody that immunologically reacts with a substance to be detected is immobilized. Immobilization of the antibody that immunologically reacts with a substance to be detected to the insoluble membrane support can be achieved by a conventionally known method. In the case of a lateral flow-based immunochromatography reagent, the antibody can be immobilized by preparing a liquid containing the antibody at a prescribed concentration, applying the liquid in a line form on the insoluble membrane support using, for example, an apparatus having a mechanism that can move a nozzle in a horizontal direction while ejecting the liquid at a constant speed from the nozzle, and drying the liquid. The liquid preferably has a concentration of the antibody of 0.1 to 5 mg/mL, and more suitably 0.5 to 2 mg/mL. The amount of an antibody immobilized on the insoluble membrane support can be optimized by adjusting the ejection speed from the nozzle of the apparatus in the case of a lateral flow format, and is suitably 0.5 to 2 µL/cm.

Note that the measurement method using a lateral flow-based immunochromatography reagent is a measurement method of a form in which a sample develops so as to move by the capillarity in a direction parallel to the insoluble membrane support.

The liquid containing an antibody at a prescribed concentration can be prepared by adding the antibody to a buffer. Examples of the type of the buffer include a phosphate buffer, Tris buffer, and Good's buffer which are generally used. The buffer preferably has a pH in the range of 6.0 to 9.5, more preferably 6.5 to 8.5, and further preferably 7.0 to 8.0. The buffer may further contain a salt, such as sodium chloride, a stabilizer or preservative, such as sucrose, an antiseptic, such as ProClin (registered trademark), or the like. The salt includes, in addition to salt that is incorporated for adjusting the ionic strength, such as sodium chloride, a salt that is added for adjusting the pH of the buffer, such as sodium hydroxide.

After immobilizing the antibody on the insoluble membrane, the portion other than the antibody-immobilized portion can further be coated with a solution or vapor of a blocking agent which is generally used for blocking.

Note that, on the insoluble membrane, a control capture reagent which is conventionally used in an immunochromatography reagent may be immobilized. The control capture reagent is a reagent for securing the reliability of the assay, and captures a control reagent incorporated in the conjugate pad. For example, when the conjugate pad contains labeled keyhole-limpet hemocyanin (hereinafter referred to as KLH) as a control reagent, an anti-KLH antibody and the like corresponds to the control capture reagent. The position where the control capture reagent is immobilized can be appropriately selected so as to comply with the design of the assay system.

Besides the above, the insoluble membrane can contain the polyoxyethylene alkyl amine in the present invention.

As a membrane constituting the insoluble membrane used in the present invention, a known membrane which has conventionally been used as an insoluble membrane support of an immunochromatography reagent can be used. Examples thereof include membranes formed of fibers of polyethylene, polyethylene terephthalate, nylons, glass, polysaccharides such as cellulose and cellulose derivatives, and ceramics. Specific examples include glass fiber filter papers and cellulose filter papers commercially available from Sartorius AG, Millipore Corp., Toyo Roshi Kaisha, Ltd., Whatman(registered trademark), or the like. Among them, UniSart CN140 from Sartorius AG is preferred. In addition, by appropriately selecting the pore size and structure of the insoluble membrane support, it is possible to control the speed of the complex of the conjugate and the substance to be detected in a sample flowing in the insoluble membrane support.

The immunochromatographic test strip is preferably disposed on a solid phase support, such as a plastic adhesive sheet. The solid phase support is configured of a substance that does not impair the capillary flow of the sample and the conjugate. The immunochromatographic test strip can be fixed with an adhesive or the like on the solid phase support. In this case, the components and the like of the adhesive are also configured of a substance that does not impair the capillary flow of the sample and the conjugate. The immunochromatographic test strip can be used in a state housed in or mounted on an appropriate container (housing) taking into account the size of the immunochromatographic test strip, the manner and position of applying a sample, the position of the detection portion formed in the insoluble membrane, and the method of detecting signals. Such a state stored or mounted is referred to as a "device".

### (Antibody to Mycoplasma pneumoniae Protein P30)

The antibody to *Mycoplasma pneumoniae* protein P30 of the present invention may be either of a polyclonal antibody or a monoclonal antibody as long as it is an antibody capable of specifically detecting the protein, and among them, a monoclonal antibody is preferred.

Such an antibody to *Mycoplasma pneumoniae* protein P30 can be obtained according to an ordinary method using, as an immunizing antigen, for example, the protein P30 extracted from *Mycoplasma pneumoniae* bacterial cells, or a recombinant protein P30 obtained by expressing a cloned protein P30 gene by genetic engineering with a host, such as E. coli, followed by extraction and purification, or a polypeptide constituting a part of the protein P30. A polyclonal antibody can be obtained from an antiserum generated when an animal, such as a mouse, is immunized with the immunizing antigen. A monoclonal antibody can be obtained by immunizing an animal, such as a mouse, then subjecting a spleen cell and a myeloma cell of the immunized animal to cell fusion, selecting and then growing the resulting fused cell in a HAT-containing medium, and screening, for example, an anti-protein P30 antibody producing strain using the protein P30.

The antibody to *Mycoplasma pneumoniae* protein P30 of the present invention also includes a fragment of antibody to *Mycoplasma pneumoniae* protein P30 and a modified antibody which are substantially equivalent to the antibody. Examples of the antibody fragment include a Fab fragment, a F(ab)₂ fragment, a Fab fragment, and an scFv fragment.

A labeled antibody which is the first antibody to *Mycoplasma pneumoniae* protein P30 of the present invention and an immobilized antibody which is the second antibody to *Mycoplasma pneumoniae* protein P30 of the present invention may each be a polyclonal antibody or a monoclonal antibody, but preferably at least one thereof is a monoclonal antibody, and more preferably, the both is a monoclonal antibody.

In addition, the first antibody and the second antibody are preferably antibodies that recognize different epitopes present in the protein P30.

The protein P30 is one of adhesion proteins having a molecular weight of 30 KDa. In bacterial cells of *Mycoplasma pneumoniae,* the protein P30 is localized on the surface of the cells at a tip of an attachment organelle, and is a transmembrane protein with the N-terminal embedded in the cell membrane and the C-terminal present outside the cell membrane.

In addition, a repetitive structure of an amino acid sequence containing many proline residues is present on the C-terminal side. In general, it is known that a region including an amino acid sequence containing many proline residues has a steric higher order structure and can be an epitope that reacts with an antibody.

The antibody to the protein P30 of the present invention is any antibody that can specifically recognize the protein P30. A preferred monoclonal antibody is a monoclonal antibody that recognizes a portion in an extracellular region of the protein P30, the portion including a repetitive structure of an amino acid sequence that contains many proline residues.

As the antibody to *Mycoplasma pneumoniae* protein P30 of the present invention, a commercially available anti-P30 monoclonal antibody may be used. Examples of the commercially available anti-P30 monoclonal antibody include C01939M, C01940M, C01941M, C01942M, and C01943M manufactured by Meridian Life Science, Inc.

### (Preparation Example of Anti-P30 Monoclonal Antibody)

The anti-P30 monoclonal antibody used in the following test was obtained by a method (method of Kohler and Milstein (Nature, Vol. 256, p.495 (1975)) and the like) which is generally used by a person skilled in the art for producing a monoclonal antibody.

### (Labeling Body)

As the labeling body used in the present invention, a known labeling body which is conventionally used in an immunochromatographic test strip can be used. For example, a colloidal metal particle, such as a colloidal gold particle or a colloidal platinum particle, a colored latex particle, a magnetic particle, a fluorescent particle, or the like is preferred, and a colloidal gold particle or a colored latex particle is particularly preferred.

### (Conjugate)

In the conjugate used in the present invention, an antibody that immunologically reacts with *Mycoplasma pneumoniae* which is a substance to be detected is immobilized to the labeling body as described above. In a preferred example of the conjugate, an anti-*Mycoplasma pneumoniae* protein P30 monoclonal antibody or polyclonal antibody is immobilized to a colloidal gold particle.

Examples of methods of immobilizing an antibody that immunologically reacts with a substance to be detected to a labeling body include physical adsorption and chemical bonding. An antibody is generally immobilized by physical adsorption.

### (Kit)

The kit for detecting *Mycoplasma pneumoniae* of the present invention may be any kit that includes a detection reagent using an antibody to *Mycoplasma pneumoniae* protein P30 and a sample diluent containing a polyoxyethylene alkyl amine.

Alternatively, the kit for detecting *Mycoplasma pneumoniae* of the present invention using immunochromatography may be any kit that includes an immunochromatographic test strip using an antibody to *Mycoplasma pneumoniae* protein P30 and a sample diluent containing a polyoxyethylene alkyl amine.

The detection kit may further include a reagent required for detection, a test tube, a sampling tool, an instruction, a housing for containing the test strip, or the like.

### (Other)

As used herein, the term "upstream" or "downstream" is used with a meaning of "on the upstream side" or "on the downstream side" in the direction of a sample flow. That is, when a sample pad, a conjugate pad, a third pad, and an insoluble membrane are stacked from above so as to partially overlap in the test strip of the present invention, the sample pad is located the most upstream and the insoluble membrane is located the most downstream. In addition, in some cases, an end pad is stacked on the insoluble membrane so that the ends thereof on the downstream side overlap with each other, and in this case, the end pad is located the most downstream.

### EXAMPLES

### [Example 1] Study on addition of surfactant to sample diluent (1)

### 1. Test materials

### (1) Test device

A test device comprising an immunochromatographic test strip using a monoclonal antibody to *Mycoplasma pneumoniae* protein P30 was produced.

In Fig. 1, a schematic configuration diagram of an immunochromatographic test strip of the present invention is illustrated.

An insoluble membrane (b) was bonded to a plastic adhesive sheet (a), then a third pad (g), a conjugate pad (d), and a sample pad (e) were placed and attached in this order, and an absorption pad (f) was placed and attached on the opposite end. The conjugate pad had been impregnated with a conjugate in which a gold colloid was sensitized with an anti-protein P30 monoclonal antibody (labeled antibody S08214R shown in the Table). On the insoluble membrane, an anti-protein P30 monoclonal antibody (immobilized antibody S08210R, application concentration: 0.75 mg/mL, application amount: 1.0 µL/cm) and a control reagent (goat anti-mouse IgG, application concentration: 0.75 mg/mL, application amount: 1.0 µL/cm) are each immobilized on a line perpendicular to the flow direction. The pads are stacked so that each pad is partially in contact with the pad above and the pad below. The line of the anti-protein P30 monoclonal antibody is referred to as a test line (c1) and a line of the control reagent is referred to as a control line (c2).

The structure in which the components were stacked was cut into a constant width to produce an immunochromatographic test strip. The test strip was mounted on and stored in a dedicated plastic housing into a form of an immunochromatography test device (not shown).

### (2) Sample diluent

To a 20 mM phosphate buffer (pH 7.5), a polyoxyethylene alkyl amine was added at 1.0% and NaCl was added at 50 mM to prepare a sample diluent. BSA was added at 0.25% as an additive.

### (3) Sample

Frozen Mycoplasma received from NBRC was concentrated by centrifugation, and was diluted with a saline solution at dilution rates in the Table. The dilutions were used as specimens. Each specimen 15 µL was suspended in 300 µL of the sample diluent to prepare a sample.

### 2. Test method

One hundred and twenty (120) µL of the sample was added dropwise to the test device and after 15 minutes, the intensity of color development of the test line was visually evaluated using a color sample called "color chart" according to the following criteria.
+: Color developed
±: Color developed but weak or very weak
-: No color developed

### 3. Test result

The results are shown in Table 1. The intensity of color development of the test line is shown in the column of "Myco" and the intensity of color development of the control line is shown in the column of "Cont". The results for a limit dilution or lower dilution rates (a range of detectable dilution rate) are hatched. The evaluation criteria for the present invention is whether the sensitivity is higher in the case of using the sample diluent of the present invention than in the case of using EMULGEN 108 (registered trademark, the same applies hereinafter) (0.5%) which showed a relatively higher sensitivity in the same test as employed in Reference Example described later.

The results show that the detection limit of EMULGEN (registered trademark, the same applies hereinafter) 108 was a dilution rate of 40 whereas the detection was possible with AMIET 105 in the present invention even at a dilution rate of 320 when *Mycoplasma pneumoniae* was detected using a sample diluent in which a polyoxyethylene alkyl amine was added.

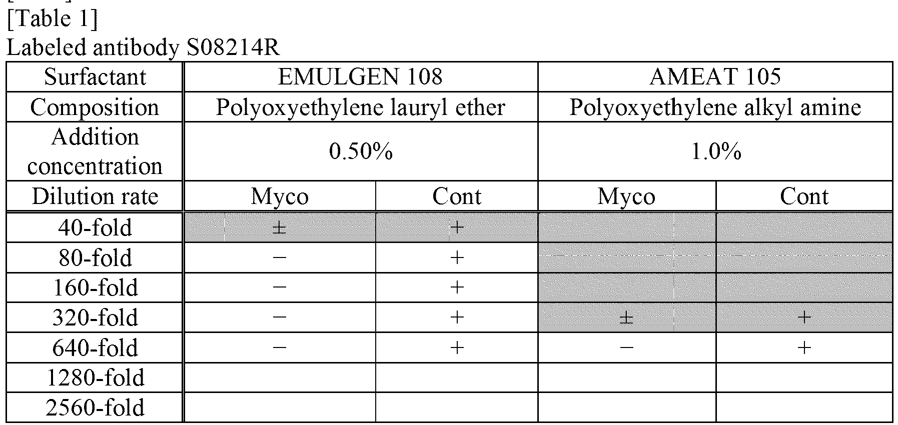

### [Example 2] Study on addition of surfactant to sample diluent (2)

The sensitivity evaluation test was carried out in the same manner as in Example 1 except for changing the labeled antibody to S08222R.

The results are shown in Table 2. The results showed that the detection limit of EMULGEN 108 was a dilution rate of 80 whereas the detection was possible with AMIET 105 in the present invention even at a dilution rate of 1280.

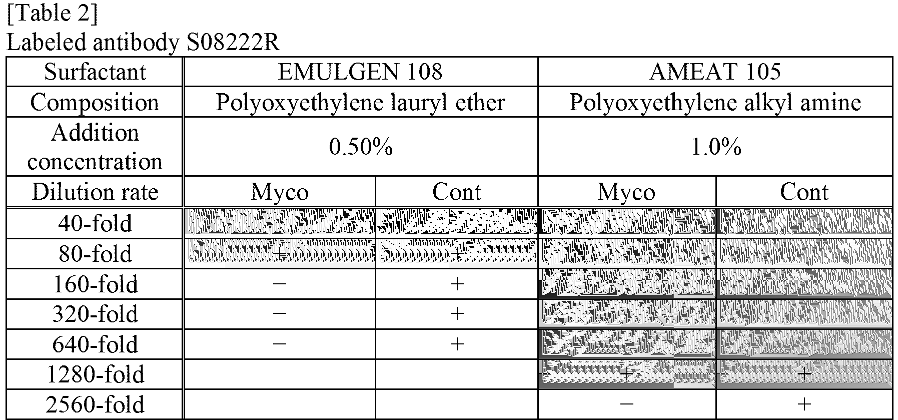

### [Example 3] Study on addition of surfactant to sample diluent (3)

The sensitivity evaluation test was carried out in the same manner as in Example 1 except for changing the labeled antibody to S08224R and changing the addition concentration of the two types of polyoxyethylene alkyl amines to 0.1%.

The results are shown in Table 3. The results showed that the detection limit of EMULGEN 108 was a dilution rate of 80 whereas the detections were possible with AMIET 105 and AMIET 320 in the present invention even at a dilution rate of 320.

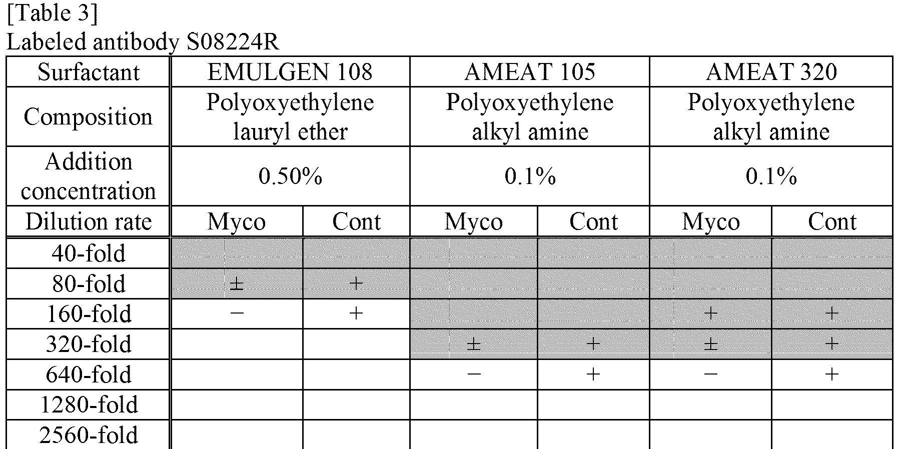

### [Example 4] Study on addition of surfactant to sample diluent (4)

The sensitivity evaluation test was carried out in the same manner as in Example 1 except for changing the labeled antibody to S08228R.

The results are shown in Table 4.

The results showed that the detection limit of EMULGEN 108 was a dilution rate of 160 whereas the detection was possible with AMIET 105 in the present invention even at a dilution rate of 640.

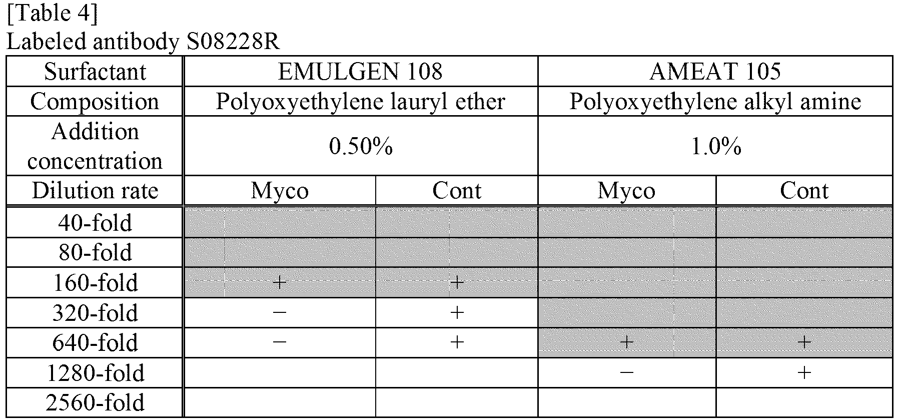

As can be seen in Examples 1 to 4 above, in any case where a monoclonal antibody to *Mycoplasma pneumoniae* protein P30 was used, *Mycoplasma pneumoniae* was able to be detected with high sensitivity by carrying out an immunoreaction in the presence of the polyoxyethylene alkyl amine in the present invention.

### [Reference Example] Screening of surfactant

Prior to Examples above, a sensitivity evaluation test was carried out for nonionic surfactants and anionic surfactants to explore a compound for increasing the detection sensitivity. While Examples 1 to 4 above evaluated the detection lower limit, this screening evaluated the intensity of color development of the test line.

### (1) Test method

One hundred and twenty (120) µL of the sample was dropwise added into the same test device as in Example 1, and after 15 minutes, the intensity of color development of the test line was visually evaluated using a color sample called "color chart". The sample diluent was adjusted by adding to a 20 mM phosphate buffer (pH 7.5) each surfactant having the composition shown in Table 5 at two concentrations of 0.1 % and 1.0 % and NaCl at 50 mM. BSA was added at 0.25% as an additive.

The evaluation criterion was a relative three-point scale as follows: with respect to the control Emargen 108, a condition in which a significant increase in the intensity of color development was observed at any of the surfactant concentration was rated at ¶, a condition in which an increase was observed was rated at §, and a condition in which a decrease was observed or color development was not observed was rated as -.

### (2) Test results

The results are shown in Table 5. The results showed that all the anionic surfactants give poor sensitivity and the detection was impossible, and among the nonionic surfactants, each of the polyoxyethylene alkyl amines gives high sensitivity. Based on these results, tests were carried out in Examples above for verifying that the detection sensitivity is also high with a plurality of additional monoclonal antibodies and for finding ranges of detectable concentrations.

**[Table 5]**

| Structure | Composition | Product name | Result of sensitivity evaluation |
|---|---|---|---|
| Nonionic surfactant | Polyoxyethylene cetyl ether | EMULGEN 210P | - |
| | Polyoxyethylene stearyl ether | EMULGEN 306P | - |
| | Polyoxyethylene stearyl ether | EMULGEN 320P | - |
| | Polyoxyethylene polyoxypropylene glycol | EPAN 410 | - |
| | | EPAN 485 | - |
| | | EPAN U-105 | - |
| | Polyoxyethylene octylphenyl ether | Triton X-100 | - |
| | Sorbitan monolaurate | LEODOL SP-L10 | - |
| | Sorbitan sesquioleate | LEODOL AO-15V | - |
| | | LEODOL TW-IS399C | |
| | Sorbitol hexa(polyoxyethylene) ether tetraoleate | LEODOL 440V | - |
| | Polyoxyethylene hydrogenated castor oil | EMANON CH-40 | - |
| | Fatty acid diester (EG/PEG ester) | LIONON DEH-40 | - |
| | Polyoxyethylene alkyl amine | AMEAT 105 | ¶ |
| | | AMEAT 320 | § |
| | Alkyl imidazoline | HOMOGENOL L-18 | - |
| | Alkyl imidazoline | HOMOGENOL L-95 | - |
| | Polyoxyethylene sorbitan monooleate | SORGEN TW-20 | - |
| | Polyoxyethylene sorbitan monooleate | SORGEN TW-80 | - |
| Anionic surfactant | Sodium alkyl sulfate | SDS | - |
| | Sodium polyoxyethylene lauryl ether sulfate | EMAL 20C | - |
| | Sodium polyoxyethylene alkyl ether sulfate | EMAL 20CM | - |
| | Sodium dialkyl sulfosuccinate | PELEX OT-P | - |
| | | PELEX TR | - |
| | Alaninate and salt thereof | ENAGICOL L-30AN | - |
| | Sodium β-naphthalene sulfonate formaldehyde condensate | DEMOL NL | - |
| | Specific carboxylic acid-type polymer surfactant | POIZ 520 | - |
| | | POIZ 530 | - |

### INDUSTRIAL APPLICABILITY

According to the present invention, by carrying out an immunoreaction using an antibody to *Mycoplasma pneumoniae* protein P30 in the presence of a polyoxyethylene alkyl amine, *Mycoplasma pneumoniae* can be detected with high sensitivity.

### REFERENCE SIGNS LIST

(a) Plastic adhesive sheet
(b) Insoluble membrane
(c1) Test line
(c2) Control line
(d) Conjugate pad
(e) Sample pad
(f) Absorption pad
(g) Third pad

## Claims

1. An immunological detection method for *Mycoplasma pneumoniae* using an antibody to *Mycoplasma pneumoniae* protein P30, wherein an immunoreaction is carried out in the presence of a polyoxyethylene alkyl amine.

2. The detection method according to Claim 1, wherein the immunological detection method is an immunochromatographic detection method.

3. The method according to Claim 1 or 2, wherein the immunochromatographic detection method includes the following steps (A) to (C):
(A) a step of mixing a sample containing *Mycoplasma pneumoniae* and a sample diluent containing a polyoxyethylene alkyl amine;
(B) a step of supplying the mixture obtained in the above (A) to the sample-supply portion of the test strip defined below,
the test strip comprising:
a membrane of a porous body having at least a sample-supply portion, a development portion, and a detection portion,
a conjugate containing a first antibody to *Mycoplasma pneumoniae* protein P30 labeled with a labeling substance retained in a part of the development portion in a dissoluble state, and
the detection portion that is located in a part of the development portion on the downstream side of the conjugate-holding portion and has a second antibody to *Mycoplasma pneumoniae* protein P30 immobilized thereon; and
(C) a step of detecting in the detection portion a complex of *Mycoplasma pneumoniae* in the sample and the conjugate.

4. A *Mycoplasma pneumoniae* detection kit comprising the following configurations (1) and (2):
(1) a detection reagent using an antibody to *Mycoplasma pneumoniae* protein P30; and
(2) a sample diluent containing a polyoxyethylene alkyl amine.

5. The detection kit according to Claim 4, wherein the detection reagent using an antibody to *Mycoplasma pneumoniae* protein P30 in the above (1) is the following configuration (1)':
(1)' an immunochromatographic test strip comprising a membrane of a porous body having at least a sample-supply portion, a development portion, and a detection portion,
a conjugate containing a first antibody to *Mycoplasma pneumoniae* protein P30 labeled with a labeling substance retained in a part of the development portion in a dissoluble state, and,
the detection portion that is located in a part of the development portion on the downstream side of the conjugate-holding portion and has a second antibody to *Mycoplasma pneumoniae* protein P30 immobilized thereon.

6. A sample diluent containing a polyoxyethylene alkyl amine for use in an immunological detection method that uses an antibody to *Mycoplasma pneumoniae* protein P30.

7. A sample pretreatment method for immunologically detecting *Mycoplasma pneumoniae,* which comprises a step of bringing a polyoxyethylene alkyl amine into contact with a sample containing *Mycoplasma pneumoniae.*
